(19) Europäisches Patentamt
European Patent Office
Office européen des brevets

(11) Veröffentlichungsnummer: **0 183 152**
**A2**

(12) # EUROPÄISCHE PATENTANMELDUNG

(21) Anmeldenummer: **85114570.6**

(22) Anmeldetag: **16.11.85**

(51) Int. Cl.⁴: **C 07 D 401/12**
**A 01 N 43/653, A 01 N 43/50**

(30) Priorität: **29.11.84 DE 3443597**

(43) Veröffentlichungstag der Anmeldung:
**04.06.86 Patentblatt 86/23**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Büchel, Karl Heinz, Prof. Dr.**
**Dabringhausener Strasse 42**
**D-5093 Burscheid(DE)**

(72) Erfinder: **Kraatz, Udo, Dr.**
**Andreasstrasse 22a**
**D-5090 Leverkusen 1(DE)**

(72) Erfinder: **Stetter, Jörg, Dr.**
**Gellertweg 4**
**D-5600 Wuppertal 1(DE)**

(72) Erfinder: **Reinecke, Paul, Dr.**
**Steinstrasse 8**
**D-5090 Leverkusen 3(DE)**

(72) Erfinder: **Brandes, Wilhelm, Dr.**
**Eichendorffstrasse 3**
**D-5653 Leichlingen(DE)**

(54) **Substituierte Azolylalkyl-pyridinyl-ether.**

(57) Die Erfindung betrifft neue substituierte Azolylalkyl-pyridinyl-ether der allgemeinen Formel

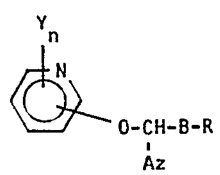

(I)

in welcher

Az, B, R und der Index n die in der Beschreibung angegebene Bedeutung besitzen, und deren Verwendung als Fungizide.

Die neuen Verbindungen werden erhalten, wenn man entsprechende Halogenetherketone mit entsprechenden Azolen umsetzt; daneben gibt es noch Synthesevarianten.

Die Verbindungen eignen sich als Pflanzenschutzmittel und können zur Bekämpfung von Pflanzenkrankheiten im Getreide- und im Reisanbau Verwendung finden.

EP 0 183 152 A2

Croydon Printing Company Ltd.

BAYER AKTIENGESELLSCHAFT 5090 Leverkusen, Bayerwerk

Konzernverwaltung RP Slr/by-c
Patentabteilung Ia

Substituierte Azolylalkyl-pyridinyl-ether

Die vorliegende Erfindung betrifft neue substituierte Azolylalkyl-pyridinyl-ether, Verfahren zu ihrer Herstellung und ihre Verwendung als Fungizide.

Es ist bereits bekannt geworden, daß Azolylalkyl-pyridinyl-ether allgemein gute fungizide Eigenschaften aufweisen (vgl. hierzu die Deutschen Offenlegungsschriften 27 56 269 , 30 00 244 und 30 28 669. Die Wirkung dieser Verbindungen ist jedoch, insbesondere bei niedrigen Aufwandmengen und -konzentrationen, nicht immer ganz befriedigend.

Es wurden neue substituierte Azolylalkyl-pyridinyl-ether der allgemeinen Formel (I)

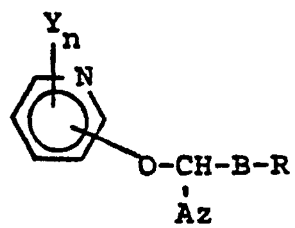

(I)

in welcher

Az für 1,2,4-Triazolyl oder für Imidazolyl steht,

B für die Keto- oder die CH(OH)-Gruppe steht,

Y für Halogen, Alkyl, Alkoxy, Alkylthio, Alkylsulfonyl, Halogenalkyl, Nitro, Cyano, Alkoxycarbonyl oder für gegebenenfalls substituiertes Phenyl steht,

R für gegebenenfalls substituiertes Aryl oder für die Gruppierungen

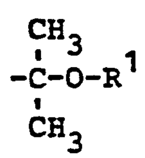 und 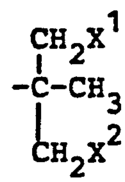 steht,

wobei

$R^1$ für gegebenenfalls substituiertes Aryl,

$X^1$ für Halogen und

$X^2$ für Wasserstoff und Halogen steht, und

n für die Zahlen 0, 1, 2 und 3 steht,

sowie deren Säureadditionssalze und Metallsalz-Komplexe gefunden.

Man erhält die substituierten Azolylalkyl-pyridinyl-ether der allgemeinen Formel (I)

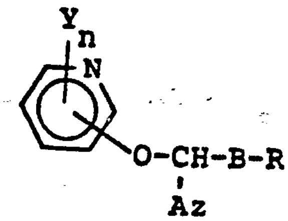

(I)

in welcher

Az für 1,2,4-Triazolyl oder für Imidazolyl steht,

B für die Keto- oder die CH(OH)-Gruppe steht,

Y für Halogen, Alkyl, Alkoxy, Alkylthio, Alkylsulfonyl, Halogenalkyl, Nitro, Cyano, Alkoxycarbonyl oder für gegebenenfalls substituiertes Phenyl steht,

R für gegebenenfalls substituiertes Aryl oder für die Gruppierungen

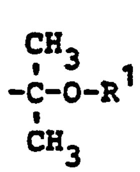 und 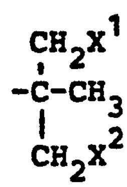 steht,

worin

$R^1$ für gegebenenfalls substituiertes Aryl,

$X^1$ für Halogen und

$X^2$ für Wasserstoff und Halogen steht, und

n für die Zahlen 0, 1, 2 und 3 steht,

wenn man Halogenetherketone der Formel (II)

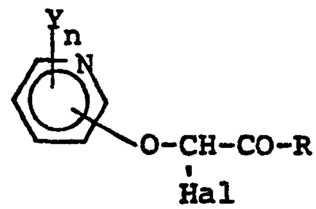

(II)

in welcher

Hal für Halogen, vorzugsweise Chlor oder Brom steht, und

Y, R und der Index n die oben angegebene Bedeutung besitzen,

mit einer Verbindung der Formel (III)

Az - H (III)

in welcher

Az die oben angegebene Bedeutung besitzt,

in Gegenwart eines Verdünnungsmittels und in Gegenwart

eines Säurebindemittels umsetzt, und gegebenenfalls noch die so erhaltenen Keto-Derivate der Formel (Ia)

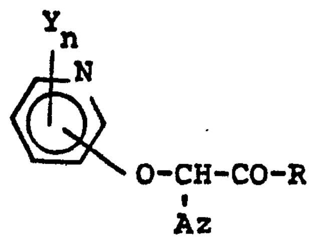

(Ia)

in welcher

Y, R, Az und der Index n die oben angegebene Bedeutung besitzen,

nach bekannten Methoden in üblicher Weise reduziert.

An die so erhaltenen Verbindungen der Formel (I) können gegebenenfalls anschließend eine Säure oder ein Metallsalz addiert werden. In manchen Fällen erweist es sich als vorteilhaft, die Verbindungen der Formel (I) über ihre Salze in reiner Form zu erhalten.

Die neuen substituierten Azolylalkyl-pyridinyl-ether der Formel (I) sowie deren Säureadditions-Salze und Metallsalz-Komplexe weisen starke fungizide Eigenschaften auf. Dabei zeigen überraschenderweise die erfindungsgemäßen Verbindungen eine bessere fungizide Wirkung als die aus dem Stand der Technik bekannten Azolylpyridinylether, die chemisch und wirkungsmäßig naheliegende Verbindungen sind.

Außerdem sind die neuen Verbindungen der Formel (I) interessante Zwischenprodukte zur Herstellung von weiteren Pflanzenschutz-Wirkstoffen. So können durch entsprechende Umsetzung funktionelle Derivate der Ketogruppe erhalten werden, wie z.B. Oxime und Oximether, Hydrazone und Ketale. Außerdem können die Verbindungen der Formel (I) an der Hydroxy-Gruppe in üblicher Weise in die entsprechenden Ether überführt werden, bzw. können durch Umsetzung mit z.B. Acylhalogeniden oder Carbamoylchloriden in prinzipiell bekannter Weise Acyl- oder Carbamoyl-Derivate erhalten werden.

Die erfindungsgemäßen Stoffe stellen somit eine wesentliche Bereicherung der Technik dar.

Die erfindungsgemäßen substituierten Azolylalkylpyridinyl-ether sind durch die Formel (I) allgemein definiert. Bevorzugt sind diejenigen Verbindungen der Formel (I), in denen

Y für Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht,

R für gegebenenfalls substituiertes Phenyl steht, wobei als Substituenten Halogen, ferner Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, ferner Nitro, Cyano und Alkoxycarbonyl mit 1 bis 4 Kohlenstoffatomen im Alkylteil, sowie gegebenenfalls durch Halogen substituiertes Phenyl zu nennen sind, und weiterhin für die Gruppierungen

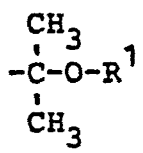 und 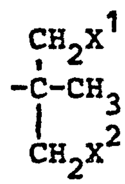 steht,

wobei

$R^1$ für Phenyl steht, welches gegebenenfalls durch die oben bei R genannten Phenylsubstituenten substituiert sein kann,

$X^1$ für Halogen und

$X^2$ für Wasserstoff und Halogen steht, und

Az, B und der Index n die in der Erfindungsdefinition genannte Bedeutung besitzen.

Besonders bevorzugt sind diejenigen Verbindungen der Formel (I), in denen

Y für Fluor, Chlor, Brom, Methyl, Isopropyl, tert.-Butyl, Methoxy, Methylthio, Methylsulfonyl, Trifluormethyl, Nitro, Cyano, Methoxycarbonyl, Ethoxycarbonyl und für gegebenenfalls durch Fluor oder Chlor ein- bis dreifach substituiertes Phenyl steht,

n für die Zahlen 0, 1 und 2 steht,

R für Phenyl steht, welches gegebenenfalls ein- bis dreifach substituiert sein kann durch Fluor, Chlor, Methyl, Ethyl, Isopropyl, tert.-Butyl, Methoxy, Methylthio, Trifluormethyl, Trifluormethoxy, Trifluormethylthio, Nitro, Cyano, sowie gegebenenfalls durch Fluor und Chlor substituiertes Phenyl, und weiterhin für die Gruppierungen

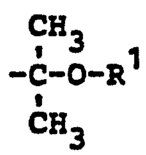 und 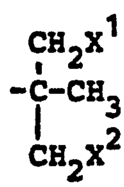 steht,

in welchen

$R^1$ für Phenyl steht, welches gegebenenfalls ein- bis dreifach substituiert sein kann durch die oben bei R genannten Phenylsubstituenten,

$X^1$ für Fluor oder Chlor und

$X^2$ für Wasserstoff, Fluor oder Chlor steht, und

Az und B die in der Erfindungsdefinition angegebene Bedeutung besitzen.

Verwendet man zur Herstellung der erfindungsgemäßen substituierten Azolylalkyl-pyridinyl-ether beispielsweise 1-Brom-3-(4-chlorphenoxy)-1-(2-chlorpyridyl-6-oxy)-3-methyl-butan-2-on und 1,2,4-Triazol als Ausgangsstoffe, so kann der Reaktionsablauf durch das folgende Formelschema wiedergegeben werden:

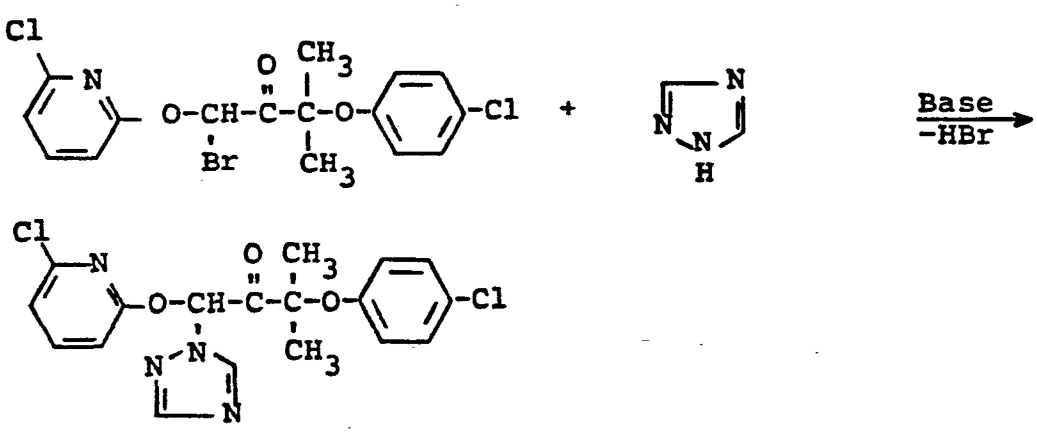


Verwendet man weiterhin zur Reduktion des im obigen Beispiel erhaltenen 1-(2-Chlorpyridyl-6-oxy)-3-methyl-3-(4-chlorphenoxy)-1-(1,2,4-triazol-1-yl)-butan-2-ons als Reduktionsmittel Natriumboranat, so kann der Reaktionsablauf durch das folgende Formelschema wiedergegeben werden:

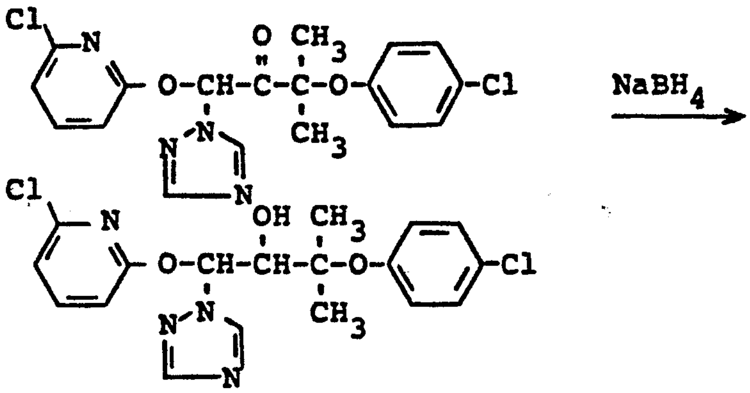


Die bei der Durchführung des erfindungsgemäßen Verfahrens zur Herstellung der Verbindungen der allgemeinen Formel (I) als Ausgangsstoffe zu verwendenden Halogenetherketone sind durch die Formel (II) allgemein definiert. In dieser Formel stehen R, Y und der Index n vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenetherketone der Formel (II) sind noch nicht bekannt. Sie können jedoch nach bekannten Verfahren erhalten werden, indem man z.B. bekannte Hydroxypyridine der Formel (IV)

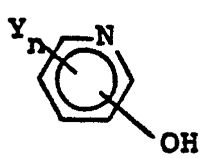

(IV)

in welcher

Y und der Index n die oben angegebene Bedeutung haben,

mit einem Halogenketon der Formel (V)

$$Hal - CH_2 - CO - R \quad (V)$$

in welcher

Hal für Chlor oder Brom steht und

R die oben angegebene Bedeutung hat,

in einem geeigneten Lösungsmittel, wie z.B. Aceton, Acetonitril oder Dioxan, im Temperaturbereich zwischen +20 und 100°C umsetzt.

Das noch verbleibende aktive Wasserstoffatom wird anschließend in üblicher Weise gegen Halogen ausgetauscht (vergleiche die Herstellungsbeispiele). Die Halogenetherketone der Formel (II) können gegebenenfalls ohne Isolierung direkt weiter umgesetzt werden.

Für die Herstellung der erfindungsgemäßen substituierten Azolylalkyl-pyridinyl-ether der allgemeinen Formel (I)

eignet sich noch eine Variante der oben angegebenen Synthese: So kann man Azolylhalogenketone der allgemeinen Formel (VI)

$$\begin{matrix} Hal - & CH & - CO - R \\ & | & \\ & Az & \end{matrix} \qquad (VI)$$

in welcher

Hal für Chlor oder Brom steht und

R und Az die oben angegebene Bedeutung besitzen,

mit den oben bezeichneten Hydroxypyridinen der allgemeinen Formel (IV) in einem geeigneten Verdünnungsmittel, wie z.B. Acetonitril, Aceton oder Dioxan, im Temperaturbereich zwischen +20 und 100°C umsetzen.

Die Azolylhalogenketone der Formel (VI) sind noch nicht bekannt. Sie können jedoch nach bekannten Verfahren erhalten werden, indem man ein Azol der Formel (III) mit einem Halogenketon der Formel (V) in einem geeigneten Verdünnungsmittel, wie z.B. Acetonitril, Aceton oder Dioxan im Temperaturbereich zwischen +20 und 100°C umsetzt und anschließend zu den Verbindungen der Formel (VI) halogeniert. Letztere können gegebenenfalls ohne Isolierung direkt weiter umgesetzt werden.

Für die erfindungsgemäße Umsetzung der Halogenetherketone der Formel (II) mit den Azolen der Formel (III) kommen als Verdünnungsmittel inerte organische Lösungsmittel in Frage.

Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Toluol; Formamide, wie insbesondere Dimethylformamid; und halogenierte Kohlenwasserstoffe.

Die erfindungsgemäße Umsetzung wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entsprechenden Überschuß an Azol.

Die Reaktionstemperaturen können in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens setzt man auf 1 Mol der Verbindung der Formel (II) vorzugsweise 2 bis 4 Mol Azol und 1 bis 4 Mol Säurebinder

ein. Zur Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemäße Reduktion der Verbindungen der Formel (Ia) erfolgt in üblicher Weise, wie z.B. durch Umsetzung mit komplexen Hydriden, gegebenenfalls in Gegenwart eines Verdünnungsmittels, oder durch Umsetzung mit Aluminium-isopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (Ia) etwa 1 Mol eines komplexen Hydrids, wie Natriumborhydrid oder Lithiumalanat, ein. Zur Isolierung der reduzierten Verbindungen wird der Rückstand in verdünnter Salzsäure aufgenommen, anschließend alkalisch gestellt und mit einem organischen Lösungsmittel

extrahiert. Die wetiere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120°C, vorzugsweise bei 50 bis 100°C.

Zur Durchführung der Reaktion setzt man auf 1 Mol des Ketons der Formel (Ia) etwa 0,3 bis 2 Mol Aluminiumisopropylat ein. Zur Isolierung der reduzierten Verbindungen wird das überschüssige Lösungsmittel im Vakuum entfernt und die entstandenen Aluminiumverbindungen werden mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie

Sulfonsäuren, wie z.B. p-Toluolsulfonsäure und 1,5-Naphthalindisulfonsäure. Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit gutem Erfolg zur Bekämpfung von Krankheiten im Getreidebau verwendet werden, so z.B. gegen Leptosphaeria nodorum, Cochliobolus sativus und Pyrenophora teres, weiterhin gegen Mehltau und Rost im Getreide. Im Reisanbau können die Wirkstoffe gegen Pyricularia oryzae und Pellicularia sasakii eingesetzt werden. Im Obstbau können die erfindungsgemäßen Wirkstoffe gegen echte Mehltaupilze, wie Podosphaera leucotricha und gegen Schorfpilze eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe,

Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalin, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid;

als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen,

Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

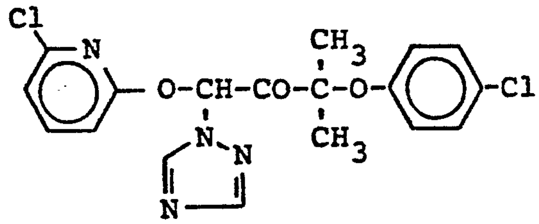

22 g (0,0052 Mol) 1-Brom-1-(2-chlorpyridyl-6-oxy)-3-(4-chlorphenoxy)-3-methyl-butan-2-on werden in 150 ml Acetonitril gelöst und mit 10,5 g (0,15 Mol) 1,2,4-Triazol versetzt. Man erhitzt die Mischung während 3 Stunden zum Sieden. Anschließend wird im Vakuum das Lösungsmittel entfernt, der Rückstand zwischen Methylenchlorid/Wasser verteilt und anschließend die organische Phase abgetrennt, getrocknet und eingeengt.

Das hinterbleibende Produkt wird zwecks weiterer Reinigung an Kieselgel in Chloroform/Essigester (4:1) chromatographiert. Man erhält 4,8 g (das sind 24 % der Theorie) 1-(2-Chlorpyridyl-6-oxy)-3-(4-chlorphenoxy)-3-methyl-1-(1,2,4-triazol-1-yl)-butan-2-on vom Fp. 100°C (aus Methanol).

Vorprodukt:

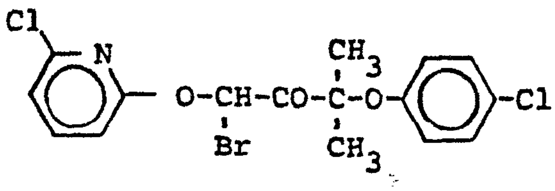

34 g (0,1 Mol) 3-Methyl-1-(2-chlorpyridyl-6-oxy)-3-(4-chlorphenoxy)-butan-2-on werden in 200 ml Tetrachlorkohlenstoff mit 17,8 g (0,1 Mol) N-Brom-succinimid versetzt und unter UV-Bestrahlung während mehrerer Stunden zum Sieden erhitzt, bis alles N-Brom-succinimid umgesetzt ist. Man filtriert ab und engt die Lösung im Vakuum ein. Das Rohprodukt wird ohne weitere Isolierung für die in Beispiel 1 angegebene Umsetzung verwendet.

Ausgangsprodukt:

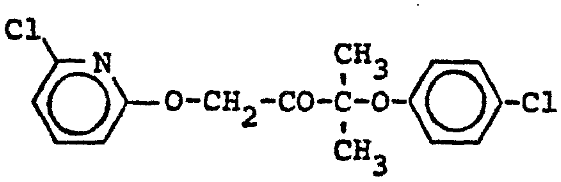

35 g (0,12 Mol) 1-Brom-3-(4-chlorphenoxy)-3-methylbutan-2-on (vgl. dazu die Angaben in EP-A 0 054 865 (Le A 20 763)) werden mit 16 g (0,12 Mol) 6-Chlor-2-hydroxy-pyridin und 28 g (0,2 Mol) Kaliumcarbonat in 300 ml Aceton während 3 Stunden zum Sieden erhitzt.

Anschließend gießt man in Wasser, extrahiert das Produkt mit Methylenchlorid und engt die organische Phase ein. Das zurückbleibende Öl wird in Chloroform über Kieselgel chromatographiert. Man erhält 34 g (das sind 83 % der Theorie) 3-(4-Chlorphenoxy)-1-(2-chlorpyridyl-6-oxy)-3-methyl-butan-2-on als wasserklares Öl.

Beispiel 1a (Strukturformel siehe Beispiel 1)

Variante des in Beispiel 1 beschriebenen Herstellungsverfahrens.

22,4 g (0,08 Mol) 3-(4-Chlorphenoxy)-3-methyl-1-(1,2,4-triazol-1-yl)-butan-2-on in 100 ml Eisessig werden bei 50°C mit 12,8 g (ca. 4,2 ml, das sind 0,08 Mol) Brom unter Rühren tropfenweise versetzt. Anschließend läßt man bis zur völligen Entfärbung nachrühren (ca. 20 Min), gießt in Wasser, extrahiert mit Methylenchlorid und wäscht die organische Phase mit Natriumhydrogencarbonat bis zur neutralen Reaktion. Danach wird unter Vakuum das Lösungsmittel entfernt und der Rückstand in 100 ml Acetonitril gelöst. Unter Rühren gibt man hierzu auf einmal eine homogene Lösung von 11,6 g (0,1 Mol) Chlorpyridin und 16 ml (0,1 Mol) Triethylamin zu. Nach Abklingen der exothermen Reaktion hält man noch während 30 Min im Sieden, gießt dann in Wasser und extrahiert mit Methylenchlorid. Die organische Phase wird zweimal mit verdünnter Natronlauge gewaschen, vom Lösungsmittel befreit und das zurückbleibende Öl aus

wenig Methanol umkristallisiert. Man erhält 12,4 g (das sind 31 % der Theorie) 1-(2-Chlorpyridyl-6-oxy)-3-(4-chlorphenoxy)-3-methyl-1-(1,2,4-triazol-1-yl)-butan-2-on vom Fp.: 101°C.

Beispiel 2

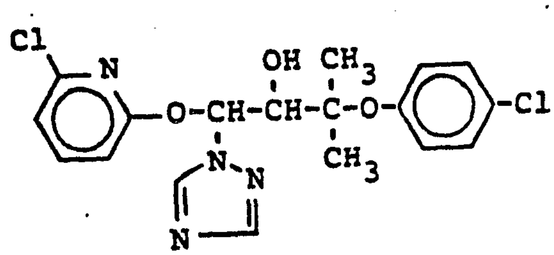

14,4 g (0,035 Mol) 1-(2-Chlorpyridyl-6-oxy)-3-(4-chlorphenoxy)-3-methyl-1-(1,2,4-triazol-1-yl)-butan-2-on (siehe Beispiel 1) werden in 100 ml Methanol mit 1 g (0,025 Mol) Natriumboranat versetzt und bei 20°C während einer Stunde gerührt. Am Rotationsverdampfer wird das Lösungsmittel entfernt und der Rückstand zwischen Methylenchlorid/Wasser verteilt. Die organische Phase wird abgetrennt, mit Wasser gewaschen und vom Lösungsmittel befreit. Man erhält 12,4 g (das sind 87 % der Theorie) an 1-(2-Chlorpyridyl-6-oxy)-3-(4-chlorphenoxy)-3-methyl-1-(1,2,4-triazol-1-yl)-butan-2-ol.

In entsprechender Weise, wie in den Beispielen 1 und 2 angegeben, werden die folgenden Verbindungen der allgemeinen Formel (Ib)

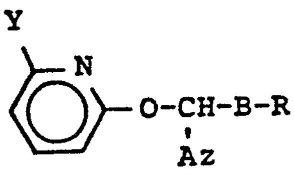

(Ib)

erhalten:

| Beispiel Nr. | Y | Az | B | R | Fp (°C) |
|---|---|---|---|---|---|
| 3 | Cl | 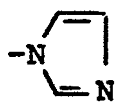 | Co | 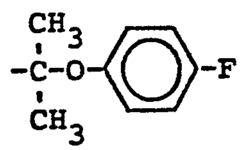 | 118 |
| 4 | Cl | 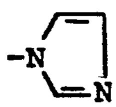 | CO | 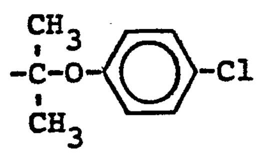 | Harz |
| 5 | Cl | 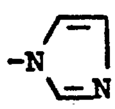 | CO | 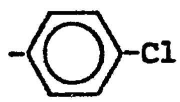 | 133 |
| 6 | Cl | 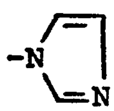 | CO | 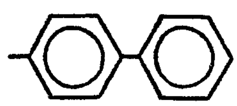 | 162 |
| 7 | F | 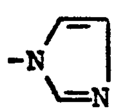 | CO | 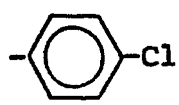 | Harz |

| Beispiel Nr. | Y | Az | B | R | Fp (°C) |
|---|---|---|---|---|---|
| 8 | Cl | 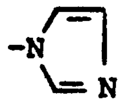 | CO | 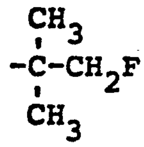 | 86 |
| 9 | F | 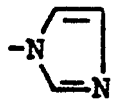 | CO | 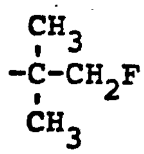 | 44-46 |
| 10 | Cl | 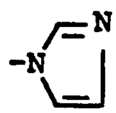 | CO | 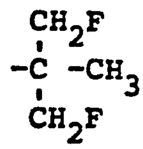 | 70 |
| 11 | F | 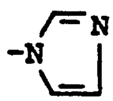 | CO | 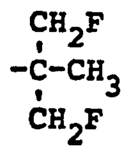 | Harz |
| 12 | Cl | 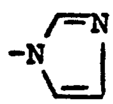 | CO | 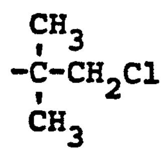 | 83 |
| 13 | F | 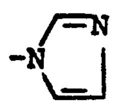 | CO | 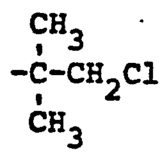 | 70-75 |
| 14 | Cl | 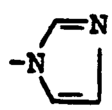 | CH(OH) | 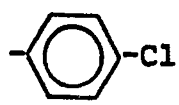 | 82 |
| 15 | Cl | 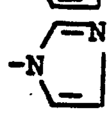 | CH(OH) | 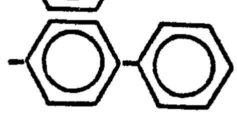 | 168 |

| Beispiel Nr. | Y | Az | B | R | Fp (°C) |
|---|---|---|---|---|---|
| 16 | F | 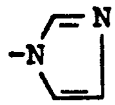 | CH(OH) | 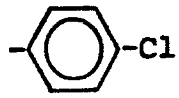 | Harz |
| 17 | Cl | 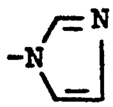 | CH(OH) | 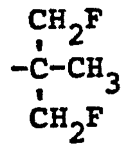 | 140 |
| 18 | F | 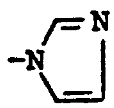 | CH(OH) | 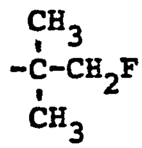 | 138-140 |
| 19 | F | 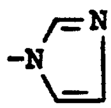 | CH(OH) | 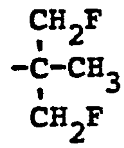 | 141 |
| 20 | Cl | 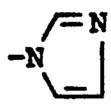 | CH(OH) | 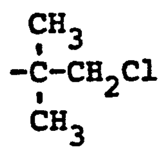 | 132-136 |
| 21 | Cl | 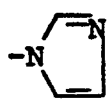 | CH(OH) | 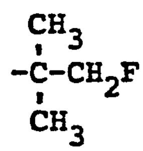 | 120-125 |
| 22 | F | 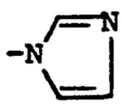 | CH(OH) | 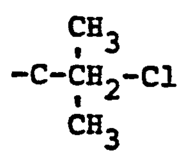 | 132 |

<u>Verwendungsbeispiele</u>

In den nachfolgenden Anwendungsbeispielen werden die nachstehend angegebenen Verbindungen als Vergleichssubstanzen eingesetzt.

(A) 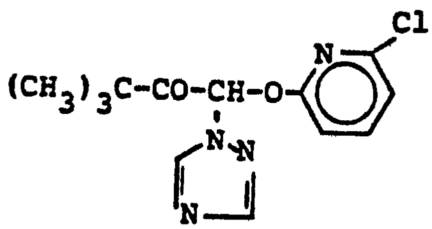

(B) 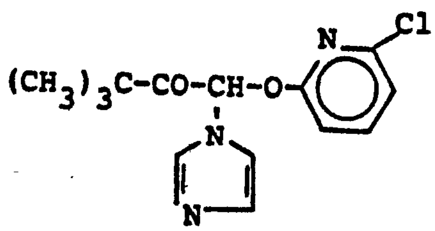

(C) 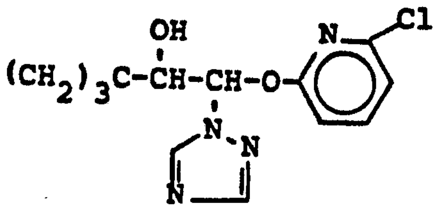

Beispiel A

Leptosphaeria nodorum-Test (Weizen) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 11, 12, 7.

Beispiel B

Podosphaera-Test (Apfel) / protektiv

Lösungsmittel: 4,7 Gew.-Teile Aceton
Emulgator: 0,3 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1,2.

Patentansprüche

1. Substituierte Azolylalkyl-pyridinyl-ether der allgemeinen Formel (I)

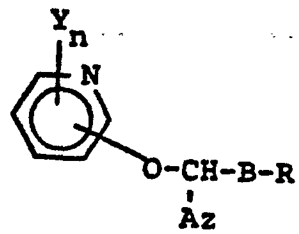

(I)

in welcher

Az für 1,2,4-Triazolyl oder für Imidazolyl steht,

B für die Keto- oder die CH(OH)-Gruppe steht,

Y für Halogen, Alkyl, Alkoxy, Alkylthio, Alkylsulfonyl, Halogenalkyl, Nitro, Cyano, Alkoxycarbonyl oder für gegebenenfalls substituiertes Phenyl steht,

R für gegebenenfalls substituiertes Aryl oder für die Gruppierungen

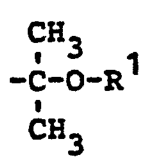 und 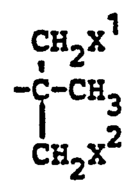 steht,

wobei

$R^1$ für gegebenenfalls substituiertes Aryl,

$X^1$ für Halogen und

$X^2$ für Wasserstoff und Halogen steht, und

n für die Zahlen 0, 1, 2 und 3 steht,

sowie deren Säureadditionssalze und Metallsalz-Komplexe.

2. Verbindungen der allgemeinen Formel (I), in welcher

Y für Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht,

R für gegebenenfalls substituiertes Phenyl steht, wobei als Substituenten Halogen, ferner Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, ferner Nitro, Cyano und Alkoxycarbonyl mit 1 bis 4 Kohlenstoffatomen im Alkylteil, sowie gegebenenfalls durch Halogen substituiertes Phenyl zu nennen sind, und weiterhin für die Gruppierungen

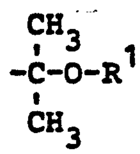 und 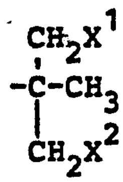 steht,

wobei

$R^1$ für Phenyl steht, welches gegebenenfalls durch die oben bei R genannten Phenylsubstituenten substituiert sein kann,

$X^1$ für Halogen und

$X^2$ für Wasserstoff und Halogen steht, und

Az, B und der Index n die in Anspruch 1 genannte Bedeutung besitzen.

3. Verfahren zur Herstellung von substituierten Azolylalkyl-pyridinyl-ethern der allgemeinen Formel (I)

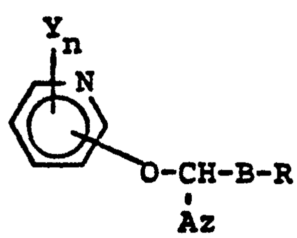

(I)

in welcher

Az für 1,2,4-Triazolyl oder für Imidazolyl steht,

B für die Keto- oder die CH(OH)-Gruppe steht,

Y für Halogen, Alkyl, Alkoxy, Alkylthio, Alkylsulfonyl, Halogenalkyl, Nitro, Cyano, Alkoxycarbonyl oder für gegebenenfalls substituiertes Phenyl steht,

R für gegebenenfalls substituiertes Aryl oder für die Gruppierungen

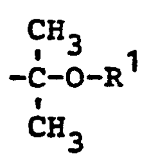 und 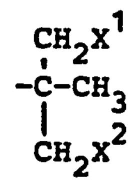 steht,

wobei

$R^1$ für gegebenenfalls substituiertes Aryl,

$X^1$ für Halogen und

$X^2$ für Wasserstoff und Halogen steht, und

n für die Zahlen 0, 1, 2 oder 3 steht,

sowie von deren Säureadditions-Salzen und Metallsalz-Komplexen,
dadurch gekennzeichnet, daß man Halogenetherketone der Formel (II)

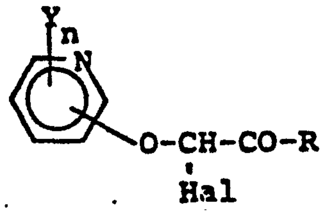

(II)

in welcher

Hal für Halogen steht, und

Y, R und der Index n die oben angegebene Bedeutung besitzen,

mit einer Verbindung der Formel (III)

Az - H (III)

in welcher

Az die oben angegebene Bedeutung besitzt,

in Gegenwart eines Verdünnungsmittels und in Gegenwart eines Säurebindemittels umsetzt, und gegebenenfalls noch die so erhaltenen Keto-Derivate der Formel (Ia)

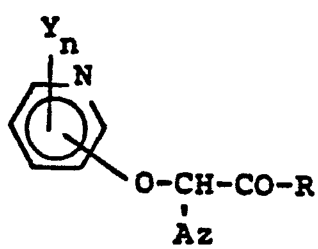

(Ia)

in welcher

Y,R,Az und der Index n die oben angegebene Bedeutung besitzen,

nach bekannten Methoden in üblicher Weise reduziert, und gegebenenfalls noch an die so erhaltenen Verbindungen der Formel (I) anschließend eine Säure oder ein Metallsalz addiert.

4. Fungizide Mittel, gekennzeichnet durch einen Gehalt an mindestens einem substituierten Azolylalkyl-pyridinyl-ether der Formel (I) bzw. an einem Säureadditions-Salz oder Metallsalz-Komplex eines substituierten Azolylalkyl-pyridinyl-ethers der Formel (I).

5. Verfahren zur Bekämpfung von Pilzen, dadurch gekennzeichnet, daß man substituierte Azolylalkyl-pyridinyl-ether der Formel (I) bzw. deren Säureadditions-Salze und Metallsalz-Komplexe auf Pilze oder ihren Lebensraum einwirken läßt.

6. Verwendung von substituierten Azolylalkyl-pyridinylethern der Formel (I) bzw. von deren Säureadditions-Salzen oder Metallsalz-Komplexen als Pflanzenschutzmittel.

7. Verwendung von substituierten Azolylalkyl-pyridinylethern der Formel (I) bzw. von deren Säureadditions-Salzen oder Metallsalz-Komplexen zur Bekämpfung von Pilzen.

8. Verfahren zur Herstellung von fungiziden Mitteln, dadurch gekennzeichnet, daß man substituierte Azolylalkyl-pyridinyl-ether der Formel (I) bzw. deren Säureadditions-Salze oder Metallsalz-Komplexe mit Streckmitteln und/oder oberflächenaktiven Mitteln vermischt.